# NOUVEAU FASCICULE DE BREVET EUROPEEN

(11) **EP 1 634 579 B2**
(45) Date de publication et mention de la décision concernant l'opposition: **13.11.2019**
(45) Mention de la délivrance du brevet: 30.11.2016
(21) Numéro de dépôt: 05291797.8
(22) Date de dépôt: 29.08.2005
(51) Int. Cl.: A61K 8/04, A61K 8/81, A61K 8/86, A61K 8/33, A61Q 5/06

(54) **Dispositif aérosol contenant au moins un polymère associatif acrylique et au moins un polymère fixant**
Aerosolbehälter enthaltend mindestens ein assoziatives Acrylpolymer und mindestens ein fixierendes Polymer
Aerosol device comprising at least an acrylic associative polymer and at least a fixing polymer

(30) Priorité: 09.09.2004 FR 0452006
(43) Date de publication de la demande: 15.03.2006
(73) Titulaire: L'Oréal, 75008 Paris (FR)
(72) Inventeur: Gringore, Charles, 75017 Paris (FR); Pataut, Françoise, 75017 Paris (FR)
(74) Mandataire: L'Oreal

(56) Documents cités:
- EP-A- 0 769 290
- EP-A- 0 797 979
- FR-A- 2 739 282
- FR-A- 2 758 334
- FR-A- 2 798 589
- US-A- 5 723 113
- US-A- 6 139 849
- US-A1- 2003 143 180
- US-B1- 6 635 240

## Description

La présente invention concerne un dispositif aérosol contenant au moins un polymère associatif acrylique et au moins un polymère fixant, ainsi qu'un procédé de coiffage utilisant un tel dispositif.

Les compositions de coiffage conditionnées sous forme de spray aérosol contiennent généralement une fraction importante d'alcool. Or, les produits cosmétiques à haute teneur en alcool font l'objet d'une surveillance particulière, notamment aux Etats Unis, suite à la sensibilisation récente de l'opinion publique aux problèmes écologiques résultant de l'émission de produits organiques volatils dans l'atmosphère.

Une solution permettant de diminuer la quantité d'alcool, voire de supprimer totalement l'alcool des formules, est le remplacement par une quantité équivalente d'eau. Cette introduction de quantités importantes d'eau dans les sprays aérosols destinés à la fixation des cheveux, tels que des laques, conduit toutefois à une modification importante indésirable de la forme de la chevelure et à une dégradation des propriétés cosmétiques. Par ailleurs, la plupart des agents propulseurs de type hydrocarbures sont incompatibles avec l'eau et leur utilisation dans des compositions à forte teneur en eau est de ce fait généralement impossible.

Les produits coiffants sont habituellement utilisés pour apporter une tenue durable à la coiffure.

Pour cela, des polymères fixants sont introduits dans un milieu hydro alcoolique.

Le conditionnement sous forme d'aérosol permet une répartition régulière sur l'ensemble de la chevelure.

La fixation de la coiffure est alors quasi-immédiate et il n'est pas aisé, voire impossible de structurer la coiffure avec les mains car les cheveux sont figés instantanément.

Le problème posé par l'invention est de fournir une composition cosmétique capillaire fixant convenablement la chevelure, procurant de bonnes propriétés cosmétiques avec, en particulier, une diminution de l'effet collant et séchant en un temps raisonnable, tout en se répartissant uniformément et rapidement sur la chevelure.

La présente invention a pour objet une composition conditionnée dans un dispositif aérosol telle que définie dans la revendication 1.

L'invention a également pour objet un procédé de coiffage consistant à vaporiser le mélange contenu dans ledit dispositif aérosol sur les cheveux mis en forme et à laisser sécher la chevelure ainsi traitée.

### COMPOSITION COIFFANTE

Tous les polymères fixants anioniques, non ioniques et leurs mélanges utilisés dans la technique peuvent être utilisés dans les compositions selon la présente demande.

Par polymère fixant, on entend au sens de la présente invention tout polymère susceptible d'apporter un maintien de la chevelure dans une forme donnée.

### POLYMERES FIXANTS

### POLYMERES FIXANTS ANIONIQUES

Les polymères fixants peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 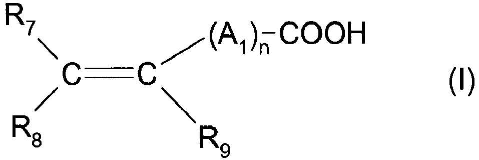 dans laquelle n est un nombre entier de 0 à 10, A₁ désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL^{®} E ou K par la société ALLIED COLLOID et ULTRAHOLD^{®} par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀, par exemple, de lauryle, tels que celui commercialisé par la société ISP sous la dénomination ACRYLIDONE^{®} LM et les terpolymères acide méthacrylique/acrylate d'éthyle/acrylate de tertiobutyle tels que le produit commercialisé sous la dénomination LUVIMER^{®} 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD^{®} DR 25 par la société AMERCHOL;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ^{®} AN ou ES par la société ISP ;
      les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne, les fonctions anhydrides de ces copolymères étant éventuellement monoestérifiées ou monoamidifiées.
      Ces polymères sont par exemple décrits dans les brevets français n^{os} 2 350 384 et 2 357 241 de la demanderesse ;
E) Les polyacrylamides comportant des groupements carboxylates.
   Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
les sels de l'acide polystyrène-sulfonique tels que les sels de sodium vendus par exemple sous la dénomination Flexan^{®} 500 et Flexan^{®} 130 par National Starch. Ces composés sont décrits dans le brevet FR 2 198 719 ; mentionnés dans le brevet US 4 128 631, et plus particulièrement l'acide polyacrylamidoéthylpropane-sulfonique vendu sous la dénomination COSMEDIA POLYMER HSP 1180 par HENKEL.

Comme autre polymère fixant anionique utilisable selon l'invention, on peut citer le polymère anionique séquencé branché vendu sous la dénomination Fixate G-100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD^{®} STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/ néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifiés vendus, par exemple, sous la dénomination GANTREZ^{®} par la société ISP, les copolymères d'acide méthacrylique et de méthacrylate de méthyle vendus sous la dénomination EUDRAGIT^{®} L par la société ROHM PHARMA, les copolymères d'acide méthacrylique et d'acrylate d'éthyle vendus sous la dénomination LUVIMER^{®} MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX^{®} A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ^{®} ES 425 par la société ISP, les terpolymères acide acrylique/acrylate d'éthyle/N-tertiobutylacrylamide vendus sous la dénomination ULTRAHOLD^{®} STRONG par la société BASF, les copolymères d'acide méthacrylique et de méthacrylate de méthyle vendus sous la dénomination EUDRAGIT^{®} L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER^{®} MAEX OU MAE par la société BASF, les terpolymères vinylpyrrolidone/acide acrylique/méthacrylate de lauryle vendus sous la dénomination ACRYLIDONE^{®} LM par la société ISP, le polymère vendu sous la dénomination Fixate G-100 par la société NOVEON.

### POLYMERES FIXANTS NON IONIQUES

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléique, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL^{®} AC-261 K et EUDRAGIT^{®} NE 30 D, par la société BASF sous la dénomination 8845, par la société HOECHST sous la dénomination APPRETAN^{®} N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH^{®} LDM 6911, MOWILITH^{®} DM 611 et MOWILITH^{®} LDM 6070 proposés par la société HOECHST, les produits RHODOPAS^{®} SD 215 et RHODOPAS^{®} DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol^{®} PLUS par la société BASF ; et
- les copolymères de vinyllactame tels qu'un copolymère poly(vinylpyrrolidone/vinyllactame) vendu sous le nom commercial Luvitec^{®} VPC 55K65W par la société BASF, les copolymères poly(vinylpyrrolidone/acétate de vinyle) comme ceux commercialisés sous la dénomination PVPVA^{®} S630L par la société ISP, Luviskol^{®} VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol^{®} VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, anioniques ou non ioniques, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 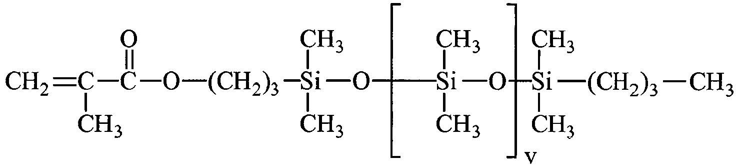 où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex^{®} Silk commercialisé par la société BASF.

La concentration en polymère(s) fixant(s) utilisée dans les compositions selon la présente invention est comprise entre 0,1 et 20%, de préférence entre 0,5 et 10% en poids par rapport au poids total de la composition totale contenue dans le dispositif aérosol (agent propulseur + composition coiffante).

### POLYMERES ASSOCIATIFS ANIONIQUES ACRYLIQUES

Au sens de la présente invention on entend par polymère associatif tout polymère amphiphile comportant au moins une chaîne grasse, donc une partie hydrophobe, et au moins une partie hydrophile.

Les molécules de ces polymères sont susceptibles de s'associer entre elles ou avec des molécules d'agents associativité comme les tensioactifs, ce qui conduit à des propriétés particulières.

La partie hydrophobe peut être en nombre réduit vis-à-vis du reste de la chaîne polymérique, et peut se situer latéralement à la chaîne et être répartie de façon aléatoire (copolymères statistiques) ou répartie sous forme de séquences ou de greffons (copolymères blocs ou copolymères séquences).

On peut utiliser des polymères solubles dans l'eau, ou hydrodispersibles. De préférence, les polymères amphiphiles utiles selon l'invention ne sont pas réticulés.

Les polymères associatifs de l'invention sont anioniques et comprennent à titre de monomères au moins un acide carboxylique insaturé. Les acides carboxyliques insaturés utilisables dans les polymères de l'invention sont de préférence choisis dans le groupe des acides acrylique, méthacrylique, crotonique, itaconique et maléique.

Par l'expression "chaîne - grasse" on doit entendre selon l'invention, un groupement hydrocarboné linéaire ou ramifiée ayant de 8 à 30 atomes de carbone.

Parmi les polymères associatifs anioniques acryliques selon l'invention, on peut citer :
- les copolymères comportant au moins un motif hydrophile, et au moins un motif éther d'allyle à chaîne grasse, dont le motif hydrophile est constitué par un acide carboxylique vinylique et tout particulièrement par un acide acrylique ou un acide méthacrylique ou les mélanges de ceux-ci, et dont le motif éther d'allyle à chaîne grasse correspond au monomère de formule (V) suivante :

   CH2 = CR'CH2OBnR (V)

   dans laquelle :
   - R' désigne H ou CH₃ ;
   - B désigne le radical éthylèneoxy ;
   - n est nul ou désigne un entier allant de 1 à 100 ;
   - R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone.

Un motif de formule (V) plus particulièrement préféré est un motif dans lequel R' désigne H, n est égal à 10 et R désigne un radical stéaryle (C₁₈).

Des polymères associatifs anioniques de ce type sont décrits et préparés, selon un procédé de polymérisation en émulsion, dans le brevet EP-0 216 479.

Parmi ces polymères, on préfère particulièrement selon l'invention les polymères formés à partir de 20 à 60 % en poids d'acide acrylique et / ou d'acide méthacrylique, de 5 à 60 % en poids de (méth)acrylates d'alkyles inférieurs, de 2 à 50 % en poids d'éther d'allyle à chaîne grasse de formule (V) et de 0 à 1 % en poids d'un agent réticulant qui est un monomère insaturé polyéthylénique copolymérisable bien connu, comme le phtalate de diallyle, le (méth)acrylate d'allyle, le divinylbenzène, le diméthacrylate de (poly)éthylèneglycol et le méthylène-bis-acrylamide.

Parmi ces derniers, on préfère tout particulièrement les terpolymères réticulés vendus par la société ALLIED COLLOIDS sous les dénominations SALCARE SC 80 ® et SALCARE SC90 ® qui sont des émulsions aqueuses à 30 % d'un terpolymère réticulé d'acide méthacrylique, d'acrylate d'éthyle et de steareth-10-allyl éther (40 / 50 / 10).

On peut aussi citer comme polymères appartenant à cette famille les produits RHOEVIS -CR, -CR3 et -CRX proposés par la société ALLIED COOLOIDS ;
- les terpolymères acide méthacrylique/acrylate d'alkyle/acrylate de lauryle polyoxyéthyléné tels que le produit RHEO 2000 commercialisé par COATEX;
- les copolymères acide méthacrylique/acrylate d'alkyle C1-C8/méthacrylate de stéaryle polyoxyéthyléné tels que les produits ACRYSOL 22, (ou ACULYN 22 ou ACRYSOL ICS) ACRYSOL25 et DW-1206A commercialisés par la société ROHM & HAAS ;
- les copolymères acides méthacrylique/acrylate d'éthyle/acrylate de nonylphénol polyoxyéthyléné tels que le produit RHEO 3000 commercialisé par COATEX;
- les terpolymères d'anhydride maléique / α-oléfine en C₃₀-C₃₈ / maléate d'alkyle tel que le produit (copolymère anhydride maléique / α-oléfine en C₃₀-C₃₈ / maléate d'isopropyle) vendu sous le nom PERFORMA V 1608 ® par la société NEWPHASE TECHNOLOGIES.
- les copolymères acryliques à motif(s) polyuréthane(s) tels que les terpolymères acryliques comprenant :
   (a) environ 20 % à 70 % en poids d'un acide carboxylique à insaturation α,β-monoéthylénique ;
   (b) environ 20 à 80 % en poids d'un monomère à insaturation α,β-monoéthylénique non-tensio-actif différent de (a) ;
   (c) environ 0,5 à 60 % en poids d'un mono-uréthane non-ionique qui est le produit de réaction d'un tensio-actif monohydrique avec un monoisocyanate à insaturation monoéthylénique ;
   tels que ceux décrits dans la demande de brevet EP-A-0 173 109 et plus particulièrement celui décrit dans l'exemple 3, à savoir, un terpolymère acide méthacrylique / acrylate de méthyle / diméthyl métaisopropényl benzyl isocyanate d'alcool béhényle éthoxylé (40 OE) en dispersion aqueuse à 25 %.

Comme produit analogue on peut citer le produit ADDITOL VXW 1312 commercialisé par HOECHST.

Parmi les polymères associatifs, on préfère :
- les terpolymères acide acrylique/acrylate d'éthyle en C1-C8 méthacrylate de stéaryle polyoxyéthyléné, par exemple à l'aide de 20 moles d'oxyde d'éthylène tels que le produit commercialisé sous la dénomination de "ACRYSOL ICS ou ACRYSOL ZZ ou ACULYN 22" par la Société ROHM & HAAS,

Selon leur nature, les polymères associatifs selon l'invention peuvent être utilisés sous forme de solutions aqueuses ou sous forme de dispersions aqueuses.

Selon leur nature, les polymères associatifs selon l'invention peuvent être utilisés sous forme de solutions aqueuses ou sous forme de dispersions aqueuses.

### AEROSOL

L'agent propulseur peut être n'importe quel gaz liquéfiable utilisé habituellement dans des dispositifs aérosol tels que le diméthyléther, les alcanes en C₃₋₅, le 1,1-difluoroéthane, les mélanges de diméthyléther et d'alcanes en C₃₋₅, et les mélanges de 1,1-difluoroéthane et de diméthyléther et/ou d'alcanes en C₃₋₅. On préfère tout particulièrement utiliser comme agent propulseur, le diméthyléther. L'agent propulseur est présent à raison de plus de 20%, et de préférence entre 20 et 70 % et encore plus préférentiellement entre 20 et 50 du poids total du mélange (agent propulseur (a) + composition coiffante (b)) contenu dans le dispositif aérosol.

Bien que les compositions de coiffage contenues dans le dispositif aérosol de la présente invention soient de préférence exemptes d'alcools inférieurs, elles peuvent contenir jusqu'à 50 % et de préférence jusqu'à 25% en poids, rapporté au poids total du mélange contenu dans le dispositif aérosol, d'un ou de plusieurs alcools inférieurs tels que l'éthanol ou l'isopropanol.

La composition coiffante selon l'invention peut contenir en outre au moins un adjuvant choisi parmi les silicones sous forme soluble, dispersée, micro-dispersée, les agents tensio-actifs non-ioniques, anioniques, cationiques et amphotères, les polymères additionnels autres que les polymères fixants et les polymères associatifs utilisés dans les compositions selon l'invention, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

Ces additifs sont présents dans la composition selon l'invention en une quantité allant de 0 à 20 % en poids par rapport au poids total de la composition.

Dans les compositions de coiffage contenues dans le dispositif aérosol, l'eau représente de 20 à 80 % en poids, rapporté au poids total du mélange contenu dans le dispositif aérosol.

Bien entendu l'homme du métier veillera à choisir ce ou ces éventuels additifs complémentaires et/ou leurs quantités de manière à ce que les propriétés avantageuses intrinsèques à l'invention ne soient pas altérées par la ou les adjonctions envisagées.

Utilisés dans les conditions de l'invention décrite ci-dessus, c'est-à-dire dans un dispositif aérosol, les polymères associatifs de l'invention facilitent l'étalement de la solution coiffante sur la chevelure et diminue l'effet collant des polymères fixants de l'invention.

Cette association particulière permet d'obtenir une mise en forme de la coiffure par un travail aux doigts sans obtenir le durcissement et le figeage instantanés classiquement rencontrés avec les produits de fixation. L'application peut se faire aussi bien sur cheveux humides que secs.

Dans le cadre d'une application sur cheveux humides un séchage à l'air libre ou un brushing peut être réalisé. Le résultat coiffure est souple et naturel.

L'introduction d'additifs spécifiques de type polyols dans ce type de formulation permet par exemple d'obtenir un effet qualifié de « wet » c'est-à-dire conférant à la chevelure une apparence type « sortie de douche » de façon durable dans le temps.

Le résultat coiffure est souple et naturel.

L'exemple de formulation illustre la présente invention sans cependant la limiter.

### Exemple :

Spray aérosol hydroalcoolique comprenant en g/100g et en matière active:
PVP : 2.27
Aculyn 22 : 0.65
2 Amino2méthyl1propanol : 0.06
Eau : 36.40
Glycérol : 10.40
Ethanol : 15.22
DME : 35

Vaporisée sur des cheveux naturels humides, la composition ci-dessus se répartit facilement sur la chevelure sans coller les doigts et lui confère après séchage une tenue souple et naturelle durable avec un effet mouillé (« wet »).

## Revendications

1. Composition conditionnée dans un dispositif aérosol, contenant
(a) au moins 20% d'au moins un agent propulseur et
(b) une composition coiffante comprenant au moins (i) un polymère fixant anionique ou non ionique non polyuréthane et (ii) au moins un polymère associatif anionique acrylique, le polymère associatif étant un polymère amphiphile comprenant au moins une chaîne grasse, c'est-à-dire un groupement hydrocarboné linéaire ou ramifié ayant de 8 à 30 atomes de carbone,
le polymère (ii) étant choisi parmi les polymères suivants :
- ceux comportant au moins un motif hydrophile, et au moins un motif éther d'allyle à chaîne grasse, dont le motif hydrophile est constitué par un acide carboxylique vinylique et tout particulièrement par un acide acrylique ou un acide méthacrylique ou les mélanges de ceux-ci, et dont le motif éther d'allyle à chaîne grasse correspond au monomère de formule (V) suivante :
CH2 = C R' CH2 O Bn R (V)
dans laquelle :
- R' désigne H ou CH₃ ;
- B désigne le radical éthylèneoxy ;
- n est nul ou désigne un entier allant de 1 à 100 ;
- R désigne un radical hydrocarboné choisi parmi les radicaux alkyle, arylalkyle, aryle, alkylaryle, cycloalkyle, comprenant de 8 à 30 atomes de carbone, de préférence 10 à 24, et plus particulièrement encore de 12 à 18 atomes de carbone.
- les terpolymères acide méthacrylique/acrylate d'alkyle/acrylate de lauryle polyoxyéthyléné;
- les copolymères acide méthacrylique/acrylate d'alkyle C1-C8 /méthacrylate de stéaryle polyoxyéthyléné,
- les copolymères acides méthacrylique/acrylate d'éthyle/acrylate de nonylphénol polyoxyéthyléné;
- les copolymères acide acrylique/monoitaconate de stéaryle polyoxyéthyléné ou les copolymères acide acrylique/monoitaconate de cétyle polyoxyéthylénés;
- les terpolymères d'anhydride maléique / α oléfine en C30-C38 / maléate d'alkyle
- les copolymères acryliques à motif(s) polyuréthane(s).

2. Composition selon la revendication précédente **caractérisée en ce que** le polymère associatif anionique acrylique est choisi parmi les terpolymères acide acrylique / acrylate d'éthyle / méthacrylate de stéaryle polyoxyéthylénée

3. Composition selon l'une quelconque des revendications précédentes, **caractérisée par le fait que** le polymère (ii) représente de 0,01 à 30%, de préférence de 0,1 à 20% et plus particulièrement de 0,5 à 15% en poids du poids total de la composition aérosol.

4. Composition selon l'une quelconque des revendications précédentes, **caractérisée en ce que** le polymère fixant anionique(i) est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, et les polymères siliconés greffés anioniques.

5. Composition selon l'une quelconque des revendications précédentes, **caractérisée en ce que** le polymère fixant non ionique (i) est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, et les polymères siliconés greffés non ioniques.

6. Composition cosmétique selon l'une des revendications précédentes telle que la concentration en polymère(s) fixant(s) (i) est comprise entre 0,1 et 20%, de préférence entre 0,5 et 10% en poids par rapport au poids total de la composition aérosol.

7. Composition selon l'une quelconque des revendications précédentes, **caractérisée par le fait que** l'eau représente de 20 à 80 % en poids, rapporté au poids total du mélange contenu dans le dispositif aérosol.

8. Composition selon l'une quelconque des revendications précédentes, **caractérisée par le fait que** l'agent propulseur (a) représente de 20 % à 70% du poids total du mélange contenu dans le dispositif aérosol, et de préférence entre 20 et 50 %.

9. Composition selon l'une quelconque des revendications précédentes, **caractérisée par le fait que** l'agent propulseur est choisi parmi le diméthyléther, le difluoroéthane, les alcanes C3 C5 et leurs mélanges.

10. Composition selon la revendication 8 **caractérisée en ce que** l'agent propulseur est le diméthyléther.

11. Composition selon l'une quelconque des revendications précédentes, **caractérisée par le fait qu'**elle contient en outre au moins un adjuvant choisi parmi les silicones sous forme soluble, dispersée, micro-dispersée, les agents tensio-actifs non-ioniques, anioniques, cationiques et amphotères, les polymères additionnels autres que les polymères fixants ou associatifs utilisés dans les compositions selon l'invention, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, les particules solides telles que par exemple les pigments minéraux et organiques, colorés ou non colorés, les agents nacrants et opacifiants, les paillettes, particules actives, les colorants, les agents séquestrants, les agents plastifiants, les agents solubilisants, les agents acidifiants, des agents alcalinisants, les agents neutralisants, les agents épaississants minéraux et organiques, les agents anti-oxydants, les hydroxyacides, les agents de pénétration, les parfums et les agents conservateurs.

12. Procédé de coiffage des cheveux consistant à vaporiser le mélange contenu dans le dispositif aérosol selon l'une quelconque des revendications précédentes sur les cheveux mis en forme et à laisser sécher la chevelure ainsi traitée.

## Patentansprüche

1. In einer Aerosolvorrichtung abgepackte Zusammensetzung, enthaltend
(a) mindestens 20% mindestens eines Treibmittels und
(b) eine Haarfrisierzusammensetzung, umfassend mindestens (i) ein anionisches oder nicht ionisches fixierendes Nicht-Polyurethan-Polymer und (ii) mindestens ein anionisches Acryl-Assoziativpolymer, wobei es sich bei dem Assoziativpolymer um ein amphiphiles Polymer mit mindestens einer Fettkette, d.h. einer linearen oder verzweigten Kohlenwasserstoffgruppe mit 8 bis 30 Kohlenstoffatomen, handelt, wobei das Polymer (ii) aus den folgenden Polymeren ausgewählt ist:
- denjenigen mit mindestens einer hydrophilen Einheit und mindestens einer Allylether-Einheit mit Fettkette, wobei die hydrophile Einheit durch eine Vinylcarbonsäure und ganz speziell durch eine Acrylsäure oder eine Methacrylsäure oder Mischungen davon gebildet wird und die Allylether-Einheit mit Fettkette dem Monomer der folgenden Formel (V) entspricht:
CH₂=CR'CH₂OBₙR (V)
in der:
- R' für H oder CH₃ steht;
- B für den Ethylenoxyrest steht;
- n für null oder eine ganze Zahl von 1 bis 100 steht;
- R für einen Kohlenwasserstoffrest steht, der aus Alkyl-, Arylalkyl-, Aryl-, Alkylaryl- und Cycloalkylresten mit 8 bis 30 Kohlenstoffatomen, vorzugsweise 10 bis 24 Kohlenstoffatomen und noch spezieller 12 bis 18 Kohlenstoffatomen ausgewählt ist;
- Terpolymeren von Methacrylsäure, Alkylacrylat und polyoxyethyleniertem Laurylacrylat;
- Copolymeren von Methacrylsäure, C1-C8-Alkylacrylat und polyoxyethyleniertem Stearylmethacrylat;
- Copolymeren von Methacrylsäure, Ethylacrylat und polyoxyethyleniertem Nonylphenolacrylat;
- Copolymeren von Acrylsäure und polyoxyethyleniertem Stearylmonoitaconat oder Copolymeren von Acrylsäure und polyoxyethyleniertem Cetylmonoitaconat;
- Terpolymeren von Maleinsäureanhydrid, C30-C38-α-Olefin und Alkylmaleat;
- Acrylcopolymeren mit einer oder mehreren Polyurethan-Einheiten.

2. Zusammensetzung nach dem vorhergehenden Anspruch, **dadurch gekennzeichnet, dass** das anionische Acryl-Assoziativpolymer aus Terpolymeren von Acrylsäure, Ethylacrylat und polyoxyethyleniertem Stearylmethacrylat ausgewählt ist.

3. Zusammensetzung nach einem der vorhergehenden Ansprüche, **dadurch gekennzeichnet, dass** das Polymer (ii) 0,01 bis 30 Gew.-%, vorzugsweise 0,1 bis 20 Gew.-% und spezieller 0,5 bis 15 Gew.-% des Gesamtgewichts der Aerosolzusammensetzung ausmacht.

4. Zusammensetzung nach einem der vorhergehenden Ansprüche, **dadurch gekennzeichnet, dass** das anionische fixierende Polymer (i) aus Homopolymeren oder Copolymeren von Acrylsäure und Methacrylsäure oder Salzen davon, Copolymeren von Crotonsäure, Copolymeren von einfach ungesättigten C₄-C₈-Carbonsäuren oder -Carbonsäureanhydriden, Polyacrylamiden mit Carboxylatgruppen, Homopolymeren oder Copolymeren mit Sulfongruppen und anionischen Silikonpfropfpolymeren ausgewählt ist.

5. Zusammensetzung nach einem der vorhergehenden Ansprüche, **dadurch gekennzeichnet, dass** das nichtionische fixierende Polymer (i) aus Polyalkyloxazolinen, Homopolymeren und Copolymeren von Vinylacetat, Homopolymeren und Copolymeren von Acrylsäureestern, Copolymeren von Acrylnitril, Homopolymeren und Copolymeren von Styrol, Polyamiden, Homopolymeren von Vinyllactam, die von Homopolymeren von Vinylpyrrolidon verschieden sind, Copolymeren von Vinyllactam und nichtionischen Silikonpfropfpolymeren ausgewählt ist.

6. Kosmetische Zusammensetzung nach einem der vorhergehenden Ansprüche, wobei die Konzentration von fixierendem Polymer bzw. fixierenden Polymeren (i) zwischen 0,1 und 20 Gew.-% und vorzugsweise zwischen 0,5 und 10 Gew.-%, bezogen auf das Gesamtgewicht der Aerosolzusammensetzung, liegt.

7. Zusammensetzung nach einem der vorhergehenden Ansprüche, **dadurch gekennzeichnet, dass** Wasser 20 bis 80 Gew.-%, bezogen auf das Gesamtgewicht der in der Aerosolvorrichtung enthaltenen Mischung, ausmacht.

8. Zusammensetzung nach einem der vorhergehenden Ansprüche, **dadurch gekennzeichnet, dass** das Treibmittel (a) 20 bis 70% des Gesamtgewichts der in der Aerosolvorrichtung enthaltenen Mischung und vorzugsweise zwischen 20 und 50% ausmacht.

9. Zusammensetzung nach einem der vorhergehenden Ansprüche, **dadurch gekennzeichnet, dass** das Treibmittel aus Dimethylether, Difluorethan, C3-C5-Alkanen und Mischungen davon ausgewählt ist.

10. Zusammensetzung nach Anspruch 8, **dadurch gekennzeichnet, dass** es sich bei dem Treibmittel um Dimethylether handelt.

11. Zusammensetzung nach einem der vorhergehenden Ansprüche, **dadurch gekennzeichnet, dass** sie außerdem mindestens ein Adjuvans enthält, das aus Silikonen in löslicher, dispergierter und mikrodispergierter Form, nichtionischen, anionischen, kationischen und amphoteren Tensiden, zusätzlichen Polymeren, die von den in den erfindungsgemäßen Zusammensetzungen verwendeten fixierenden Polymeren bzw. Assoziativpolymeren verschieden sind, Ceramiden und Pseudoceramiden, Vitaminen und Provitaminen, darunter Panthenol, pflanzlichen, tierischen, mineralischen und synthetischen Ölen, Wachsen, die von Ceramiden und Pseudoceramiden verschieden sind, silikonhaltigen oder nicht silikonhaltigen, wasserlöslichen und fettlöslichen Lichtschutzmitteln, festen Teilchen wie beispielsweise farbigen oder farblosen anorganischen und organischen Pigmenten, Glanz- und Trübungsmitteln, Pailletten, Wirkstoffteilchen, Farbmitteln, Sequestriermitteln, Weichmachern, Lösungsvermittlern, Ansäuerungsmitteln, Alkalinisierungsmitteln, Neutralisationsmitteln, anorganischen oder organischen Verdickungsmitteln, Antioxidantien, Hydroxysäuren, Penetriermitteln, Duftstoffen und Konservierungsmitteln ausgewählt ist.

12. Haarfrisierverfahren, das darin besteht, dass man die in der Aerosolvorrichtung enthaltene Mischung nach einem der vorhergehenden Ansprüche auf die in Form gebrachten Haare aufsprüht und das so behandelte Haar trocknen lässt.

## Claims

1. Composition packaged in an aerosol device, containing:
(a) at least 20% of at least one propellant; and
(b) a hairstyling composition comprising at least (i) a non-polyurethane anionic or non-ionic fixative polymer and (ii) at least one anionic acrylic associative polymer, the associative polymer being an amphiphilic polymer comprising at least one fatty chain, i.e. a linear or branched hydrocarbon group containing 8 to 30 carbon atoms,
polymer (ii) being selected from the following polymers:
- those comprising at least one hydrophilic motif and at least one fatty chain allyl ether motif the hydrophilic motif of which is constituted by a vinyl carboxylic acid and more particularly by an acrylic acid or a methacrylic acid or mixtures thereof, and the fatty chain allyl ether motif of which corresponds to the monomer with the following formula (V):
CH₂=CR'CH₂OBₙR (V)
in which:
- R' designates H or CH₃;
- B designates the ethyleneoxy radical;
- n is zero or designates a whole number from 1 to 100;
- R designates a hydrocarbon radical selected from alkyl, arylalkyl, aryl, alkylaryl, cycloalkyl radicals containing 8 to 30 carbon atoms, preferably 10 to 24, and more particularly 12 to 18 carbon atoms;
- methacrylic acid/alkyl acrylate/polyoxyethyenated lauryl acrylate terpolymers;
- methacrylic acid/C₁-C₈ alkyl acrylate/polyoxyethylenated stearyl methacrylate copolymers;
- methacrylic acid/ethyl acrylate/polyoxyethylenated nonylphenol acrylate copolymers;
- acrylic acid/polyoxyethylenated stearyl mono-itaconate copolymers or acrylic acid/ polyoxyethylenated cetyl mono-itaconate copolymers;
- maleic anhydride/C₃₀-C₃₈ α-olefin/alkyl maleate terpolymers
- acrylic copolymers with polyurethane motif(s).

2. Composition according to the preceding claim, **characterized in that** the anionic acrylic associative polymer is selected from acrylic acid/ethyl acrylate/polyoxyethylenated stearyl methacrylate terpolymers.

3. Composition according to any one of the preceding claims, **characterized in that** polymer (ii) represents 0.01% to 30%, preferably 0.1% to 20% and more particularly 0.5% to 15% by weight of the total aerosol composition weight.

4. Composition according to any one of the preceding claims, **characterized in that** the anionic fixative polymer (i) is selected from homopolymers or copolymers of acrylic and methacrylic acid or their salts, copolymers of crotonic acid, copolymers of monounsaturated C₄-C₈ carboxylic acids or anhydrides, polyacrylamides containing carboxylate groups, homopolymers or copolymers containing sulfonic groups, and anionic graft silicone polymers.

5. Composition according to any one of the preceding claims, **characterized in that** the non-ionic fixative polymer (i) is selected from polyalkyloxazolines, homopolymers and copolymers of vinyl acetate, homopolymers and copolymers of acrylic esters, copolymers of acrylonitrile, homopolymers and copolymers of styrene, polyamides, homopolymers of vinyl lactame other than homopolymers of vinylpyrrolidone, copolymers of vinyl lactame and non-ionic graft silicone polymers.

6. Cosmetic composition according to one of the preceding claims, in which the concentration of fixative polymer (s) (i) is in the range 0.1% to 20%, preferably in the range 0.5% to 10% by weight with respect to the total aerosol composition weight.

7. Composition according to any one of the preceding claims, **characterized in that** the water represents 20% to 80% by weight with respect to the total weight of the mixture contained in the aerosol device.

8. Composition according to any one of the preceding claims, **characterized in that** the propellant (a) represents 20% to 70% of the total weight of the mixture contained in the aerosol device, preferably in the range 20% to 50%.

9. Composition according to any one of the preceding claims, **characterized in that** the propellant is selected from dimethylether, difluoroethane, C₃ - C₅ alkanes and mixtures thereof.

10. Composition according to Claim 8, **characterized in that** the propellant is dimethylether.

11. Composition according to any one of the preceding claims, **characterized in that** it also contains at least one adjuvant selected from silicones in the soluble, dispersed, micro-dispersed form, non-ionic, anionic, cationic and amphoteric surfactants, additional polymers other than the fixing or associative polymers used in the compositions of the invention, ceramides and pseudo-ceramides, vitamins and pro-vitamins including panthenol, vegetable, animal, mineral and synthetic oils, waxes other than ceramides and pseudo-ceramides, hydrosoluble and liposoluble, silicone or non silicone sunscreens, solid particles such as, for example, mineral and organic pigments, which may or may not be coloured, pearlizing agents and opacifying agents, glitter, active particles, colouring agents, sequestrating agents, plasticizers, solubilizing agents, acidifying agents, alkalinizing agents, neutralizing agents, mineral and organic thickening agents, anti-oxidants, hydroxy acids, penetrating agents, fragrances and preservatives.

12. Hairstyling method consisting of spraying the mixture contained in the aerosol device according to any one of the preceding claims onto the shaped hair and allowing the thus treated hairstyle to dry.
